Europäisches Patentamt

European Patent Office

Office européen des brevets

(19)

(11) Numéro de publication: **0 001 523**
**B1**

(12) **FASCICULE DE BREVET EUROPEEN**

(21) Numéro de dépôt: 78400108.3

(22) Date de dépôt: 22.09.78

(51) Int. Cl.³: **A 61 B 6/02, A 61 B 6/06, G 21 K 1/02, G 21 K 1/10**

(54) Procédé et appareil de tomographie axiale transverse

(30) Priorité: 11.10.77 FR 7730533

(43) Date de publication de la demande: 18.04.79 Bulletin 79/08

(45) Mention de la délivrance du brevet: 03.09.80 Bulletin 80/18

(84) Etats contractants désignés: DE GB NL SE

(56) Documents cités:
DE - A - 2 548 531
DE - B - 1 079 448
FR - A - 2 019 823
FR - A - 2 329 132

(73) Titulaire: Compagnie Générale de Radiologie
13 square Max-Hymans
F - 75741 Paris Cedex 15 (FR)

(72) Inventeur: d'Haenens, Jean-Pierre
"Thomson-Csf"—Scpi 173, bld Haussmann
F - 75360 Paris Cedex 08 (FR)

(74) Mandataire: Pierre, Michel
"Thomson-Csf"—Scpi 173, boulevard Haussmann
F - 75360 Paris Cedex 08 (FR)

Courier Press, Leamington Spa, England.

## Procédé et appareil de tomographie axiale transverse

La présente invention concerne un procédé de tomographie axiale transverse ou tomo-densitomètrie, destiné à l'examen d'une section transversale d'un corps de patient à l'aide d'une ou plusieurs sources de rayonnement X émettant chacune un faisceau en éventail situé dans le plan de la section à examiner, en direction d'une pluralité de détecteurs de ce rayonnement pour déterminer l'absorption, par cette section du corps qui est située entre la source et les détecteurs, des rayons selon les différentes incidences pour obtenir, à l'aide d'un ordinateur, une image des coefficients d'absorption ou des densités respectifs des régions (surfaces) élémentaires composant cette section, et un appareil pour la mise en oeuvre de ce procédé.

Dans les appareils de tomographie axiale transverse ou tomodensitomètres connus de ce genre, du type utilisant un faisceau de rayons X en forme d'éventail plat et une série de détecteurs accolés ou juxtaposés (scintillateurs avec photomultiplicateurs, chambres d'ionisation etc.), formant une rangée droite ou courbe (en arc de cercle), la largeur des pinceaux de rayonnement reçus par ces détecteurs est généralement définie par des collimateurs dits secondaires placés en amont de ceux-ci, et en aval du corps à examiner. C'est cette largeur des pinceaux de rayonnement qui détermine la fonction de transfert de modulation du système, c'est-à-dire sa résolution.

Dans la demande de brevet français N° d'Enregistrement National 69—29050, déposée le 25 Août 1969 et mise à la disposition du public le 3 Juillet 1970 avec le N° de Publication 2.019.365, l'un des modes de réalisation décrits et illustrés comprend, outre la rangée de détecteurs et de collimateurs secondaires associés, un jeu de collimateurs primaires en forme de tubes divergents, disposés entre la source de rayons X et le corps à examiner et qui permettent de réduire l'irradiation inutile du patient en divisant le faisceau en éventail en des pinceaux discrets plus étroits, de largeurs adaptées à l'ouverture des collimateurs secondaires. Dans la demande de brevet français N° d'Enregistrement National 76—31420, déposée le 19 Octobre 1976 et mise à la disposition du public le 20 Mai 1977, avec le N° de Publication 2.329.132, on a également décrit un collimateur primaire dit radial, disposé entre la source de rayons et l'objet à examiner, et destiné à transformer le faisceau unique de rayons X en forme d'éventail plan, en un groupe de plusieurs petits faisceaux ayant chacun une profondeur (largeur) d'environ un centimètre et une épaisseur de 1,2 millimètres environ (il est présumé que ces dimensions sont pris au niveau du collimateur primaire), avec un effet analogue.

Dans les deux cas précités, les collimateurs primaires sont présents en permanence sur la trajectoire du faisceau et donc fixés solidairement sur le même support que la source, les détecteurs et les collimateurs secondaires associés à ces derniers, les mesures d'absorption y sont donc effectuées avec une résolution unique bien définie que l'on ne peut pas changer dans un appareil de tomographie axiale transverse donné.

Dans le procédé classique, on utilise des pinceaux larges ce qui permet d'obtenir un plus grand nombre de photons au niveau des détecteurs et, par conséquent, une meilleure résolution en ce qui concerne la densité dont l'absorption est une fonction, c'est-à-dire une meilleure différentiation entre valeurs proches de l'absorption (ou des densités voisines dans l'objet). Ceci est intéressant surtout pour la détection de tumeurs à leur début, par exemple, où il est nécessaire de discerner des faibles accroissements de la densité dans un tissu mou normalement relativement peu absorbant (cerveau, poumons, foie, etc.). Ce procédé présente également d'autres avantages, tels qu'une utilisation optimale de la dose de rayonnement émis et une certaine insensibilité aux déplacements relatifs du foyer du tube radiogène par rapport aux détecteurs.

Par contre l'expérience a montré que l'utilisation suivant l'invention de pinceaux étroits dont les largeurs au niveau du collimateur secondaire sont inférieures à l'ouverture de celui-ci, permet d'obtenir une meilleure résolution spatiale, ce qui est intéressant pour l'examen d'objets présentant des détails très contrastés (des structures osseuses), c'est-à-dire de fortes variations ou gradients de densité, où l'importance de la quantité de photons X reçus par les détecteurs est moindre. Toutefois, dans ce cas le nombre de photons reçus par chaque détecteur peut devenir suffisamment faible pour donner naissance à un bruit quantique plus élevé que dans le premier cas.

Le procédé et l'appareil de tomographie axiale transverse, objets de l'invention, permettent d'obtenir sélectivement une bonne résolution en densité ou une bonne résolution spatiale en utilisant un collimateur ou grille primaire escamotable que l'on peut disposer entre la source et le corps à examiner, rendant ainsi possible l'utilisation du même appareil pour les deux types de procédés de mesure soit successivement, soit séparément.

Dans le cas où la largeur des pinceaux de rayonnement est réduite au moyen de la grille primaire et du fait que la largeur des collimateurs secondaires reste constante, on obtient l'avantage que ces pinceaux étroits ont largement la place pour passer entre les parois du collimateur secondaire, d'où une meilleure tolérance aux déplacements accidentels sans bruit notable.

Suivant l'invention, un procédé de tomographie axiale transverse dans lequel une source de rayons émet un faisceau ou une pluralité de faisceaux en éventail, obtenus à l'aide d'un collimateur primaire, vers le corps à examiner, les rayons transmis étant ensuite recueillis, à travers une première rangée de collimateurs secondaires à large ouverture, par une pluralité de détecteurs formant une seconde rangée, est principalement caractérisé par le fait que, pour obtenir dans certain cas une image du corps présentant une résolution spatiale accrue, on interpose entre la source et le corps une grille primaire amovible, munie de fentes laissant passer chacune un pinceau fin de largeur inférieure à celle définie par les ouvertures de collimateurs primaires et ou secondaires et pour obtenir dans d'autres cas, une image présentant une résolution en densité accrue, on escamote la grille amovible de la trajectoire du faisceau en éventail.

Suivant un autre aspect de l'invention, un appareil de tomographie axiale transverse pour la mise en oeuvre du procédé ci-dessus, est principalement caractérisé par le fait qu'il comporte, en amont du corps à examiner, une grille primaire escamotable pouvant être insérée et extraite de la trajectoire du ou des faisceaux en éventail, la grille étant munie de fentes laissant passer une pluralité de pinceaux de rayons X dont la largeur est inférieure à celle obtenue à l'aide des collimateurs primaires et/ou secondaires.

L'invention sera mieux comprise et d'autres de ses caractéristiques et avantages ressortiront de la description ci-après et de dessins annexés s'y rapportant, donnés à titre d'exemple, sur lesquels:

— la figure 1 représente une vue partielle, très schématique en élévation frontale d'un appareil de tomodensitométrie permettant la mise en oeuvre du procédé suivant l'invention;

— la figure 2 montre une vue latérale schématique du mécanisme d'escamotage de la grille primaire; et

— la figure 3 représente plus en détail et en perspective la grille primaire escamotable avec ses mécanismes d'escamotage et de translation.

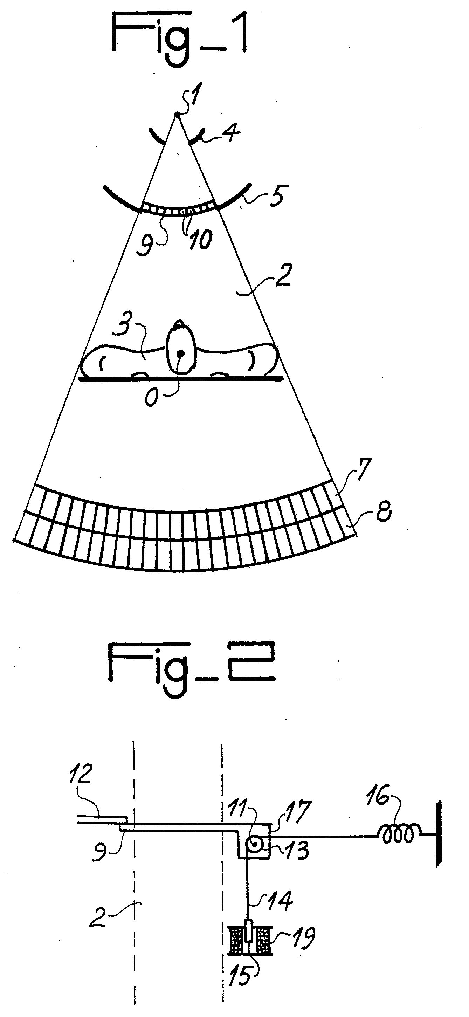

Sur la figure 1, l'appareil de tomographie axiale transverse représenté comporte une source de rayons X, tel qu'un tube radiogène classique avec sa gaine de protection munie d'une fenêtre permettant le passage du faisceau dans une direction prédéterminée, dont on a représenté le foyer qui constitue l'origine du rayonnement, par le repère 1.

Cette source de rayons X émet vers l'extérieur un faisceau en forme d'éventail plat dont les dimensions, c'est-à-dire son angle d'ouverture et son épaisseur, sont définies au moyen de deux diaphragmes primaires 4 et 5 formant ensemble un système dit de collimateurs primaires, car il se trouve disposé en amont de l'objet à examiner, à proximité de la source.

Les deux diaphragmes primaires 4 et 5 sont représentés sur la figure 1 comme des plaques métalliques de forme cylindrique avec leur centre de courbure situé dans le foyer 1, respectivement munis de fentes de forme allongée dont la largeur relativement peu importante définit l'épaisseur du faisceau, et dont les longueurs respectives (sensiblement homothétiques par rapport au foyer 1) déterminent l'angle d'ouverture de l'éventail. Les diaphragmes primaires 4, 5 peuvent également être réalisés à l'aide de plaques planes avec des résultats analogues.

Il est à remarquer ici qu'il est possible de remplacer les diaphragmes primaires 4, 5 ou la seconde plaque à fente transversale 5, par un collimateur primaire découpant le faisceau unique en plusieurs faisceaux situés dans le même plan de coupe, ayant une ouverture angulaire (largeur) adaptée à celle de l'ouverture des collimateurs secondaires 7 qui seront décrits plus loin, de façon à réduire l'irradiation du patient.

L'objet à radiographier constitué par le corps 3 d'un patient reposant sur un support transparent aux rayons X (non représenté) est disposé dans le champ du faisceau en éventail de sorte que son axe longitudinal soit parallèle à l'axe de rotation O de l'appareil et que l'organe à examiner soit approximativement centré sur cet axe de l'appareil dont font partie la source 1 et le collimateur 4, 5.

En aval de l'objet 3, c'est-à-dire du côté opposé à celui de la source 1, est placé un ensemble composé d'une rangée de détecteurs juxtaposés 8 (assemblages de scintillateurs avec des photomultiplicateurs, chambres d'ionisation ou tout autre dispositif traduisant l'intensité reçu des rayons X en un signal électrique), précédée d'une rangée de collimateurs secondaires 7 (situés entre l'objet 3 et les détecteurs 8) focalisés sur le foyer 1 de la source de rayons X. Les rangées respectives de détecteurs 8 et de collimateurs 7, ont été représentées ici comme étant disposées sur des arcs de cercle concentriques, leurs centres de courbure coïncidant avec le foyer 1.

La source 1 avec les collimateurs primaires 4, 5 ainsi que les rangées de détecteurs 8 et de collimateurs secondaires 7, sont rendues solidaires par montage direct ou indirect sur un support rotatif (non représenté), en forme de couronne dont le centre est situé sur son axe de rotation O.

Un tel appareil est utilisable pour la tomodensitométrie en un premier mode donnant une bonne résolution en densité, puisque les pinceaux élémentaires de rayonnement frappant chacun des détecteurs de la rangée 8 en provenance de l'objet 3, sont définis par les collimateurs secondaires 7 et, de ce fait, relativement larges.

Ce premier mode d'exploitation, dit de résolution en densité, est particulièrement adapté à détecter des faibles variations de la densité sur des zones élémentaires voisines et donne des images avec des transitions graduelles puisque les mesures intègrent une section relativement importante.

Pour détecter de fortes variations de la densité dans l'objet sur de relativement faibles distances, il sera nécessaire de limiter la largeur des pinceaux de rayons pour obtenir une meilleure résolution spatiale au prix d'une résolution en densité plus faible. Ceci est obtenu, dans un second mode d'exploitation dit de résolution spatiale, à l'aide d'une grille primaire 9 que l'on dispose sur la trajectoire du faisceau en éventail, par exemple à proximité du second étage 5 du collimateur primaire 4, 5.

Cette grille 9 est, de préférence, en arc de cercle ayant son centre de courbure confondu avec le foyer 1 de la source de rayons X, sa longueur dépassant légèrement celle de la fente du second étage 5, qu'elle obture en partie. La grille 9 comporte une pluralité de fentes rectangulaires allongées 10 qui sont juxtaposées et dont les centres sont homothétiques par rapport au foyer 1 avec ceux des collimateurs secondaires 7. Elle peut être constituée de tout matériau à nombre atomique élevé absorbant les rayons X tel que, par exemple, une plaque de plomb perforée de fentes minces rectangulaires ou plusieurs lamelles de plomb superposées, perforées de fentes minces rectangulaires et réunies ensemble par un matériau transparent aux rayons X (résines). Il est avantageux, à cause de son usinage relativement aisé, d'utiliser une grille 9 fabriquée à partir d'une plaque de tantale fraisée.

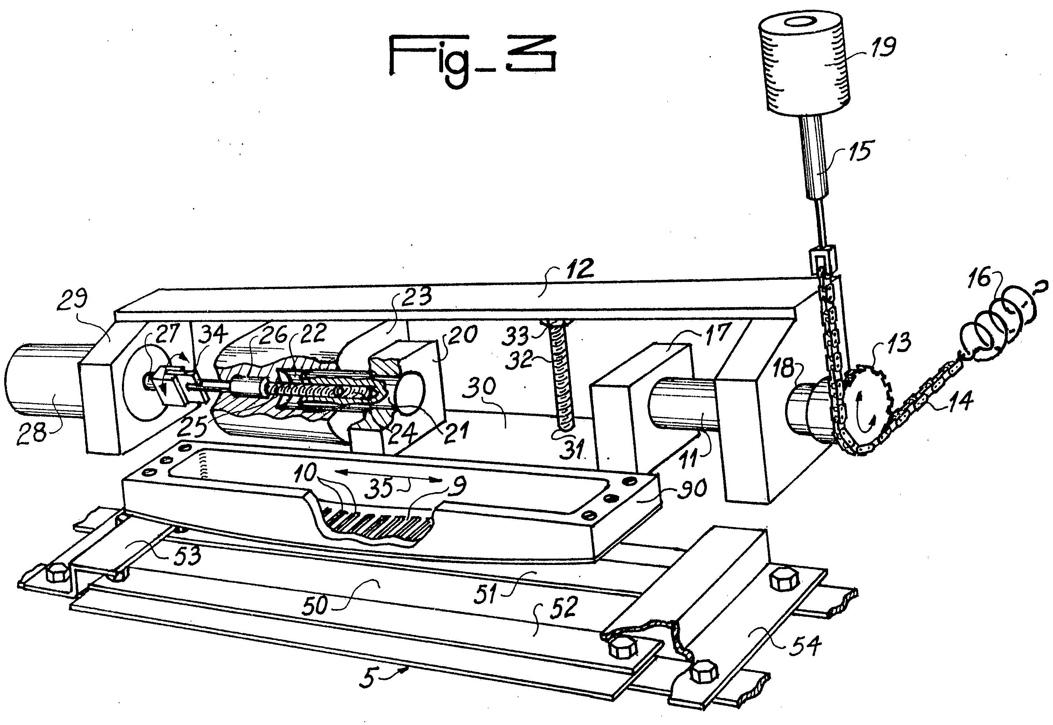

La figure 2 montre schématiquement une vue latérale en élévation d'un mode de réalisation du mécanisme d'escamotage de la grille 9 de façon à permettre l'exploitation du tomodensitomètre de la figure 1 en mode de résolution en densité ou spatiale. Sur les figures 2 et 3 on a désigné les mêmes éléments par les mêmes chiffres.

La grille primaire 9 est montée ici sur un support 17 solidaire d'un axe de pivotement 11 monté de façon pivotable dans un logement 18 (figure 3), solidaire du bâti. L'axe 11, qui est ici parallèle au plan du faisceau en éventail et sensiblement perpendiculaire au rayon central de celui-ci, porte en bout un pignon 13 qui engrène avec une chaîne 14 tendue en équerre autour de celui-ci, dont une extrémité est fixée à un moyen élastique 16, tel qu'un ressort de rappel ancré sur le bâti, et dont l'autre extrémité est fixée sur le noyau plongeur 15 d'un électro-aimant 19 également fixé au bâti.

L'électro-aimant 19 est du type bistable et il est commandé par des moyens de commande permettant la sélection du mode d'utilisation de l'appareil (non représentés). Ces moyens commandent en même temps l'ordinateur pour qu'il modifie les paramètres de calcul de l'image de la section transversale reconstituée suivant que la grille 9 est présente ou absente sur la trajectoire du faisceau 2. Une butée 12 (en forme de plaque) fixe permet de positionner la grille 9 correctement pour qu'elle soit orientée perpendiculairement au faisceau 2.

Il est à noter ici que d'autres types de mécannismes d'escamotage sont envisageables, tels que le pivotement à l'aide d'un moteur électrique couplé par engrenages à l'axe 11, translation latérale, par exemple, perpendiculairement au rayon central du faisceau ou pivotement autour d'un axe parallèle au plan de la figure 2.

Sur la figure 3, la grille primaire 9 a été représentée, avec les mécanismes de son pivotement et de sa translation, en perspective en utilisant les mêmes chiffres de repère que dans les figures 1 et 2 pour désigner les mêmes éléments.

En bas de la figure 3, on a représenté le second étage 5 du collimateur primaire 4, 5 qui comporte une fente 50 rectangulaire de forme allongée laissant passer le faisceau de rayons X en forme d'éventail. La fente 50 est bordée latéralement par deux longerons 51, 52 dont l'espacement relatif permet de déterminer l'épaisseur du faisceau, et aux deux bouts par deux pièces profilées 53, 54 définissant son ouverture angulaire, qui ont été symbolisées sur la figure 1 par des traits en arcs de cercles.

Au dessus du cadre 5, l'on aperçoit la grille primaire 9 formant une surface cylindrique en tantale fraisé, munie d'une rangée de fentes allongées 10, orientées parallèlement à la génératrice de la surface et disposées sur celle-ci de façon à pouvoir être alignées avec les zones centrales respectives des collimateurs secondaires 7.

La grille 9 est montée sur un cadre rigide 90 qui est solidaire de deux supports 17 et 20 en forme de blocs. Le premier bloc de support 17 est solidaire du premier axe 11 logé dans un palier 18 fixé au bâti de l'appareil, de façon rotative et déplaçable axialement. L'extrémité libre de l'axe 11 porte le pignon 13 qui engrène avec la chaîne 14 du mécanisme d'escamotage déjà schématisé sur la figure 2.

Le second bloc de support 20 porte solidairement un second axe 21 en saillie du côté opposé à celui du premier axe 11, et qui s'insère dans un second palier 22 en forme de trou pratiqué dans un bloc métallique 23 monté fixe sur le bâti tournant de l'appareil de tomodensitométrie.

Le second axe 21 comporte un filetage axial interne 24 de façon à former un écrou dans lequel est engagé un arbre fileté 25 passant par un second palier ou logement 26 du bloc 23 et couplé à l'arbre 27 d'un moteur électrique 28 dont le stator est fixé au bâti à l'aide d'un bloc de montage 29.

Le cadre 90 et les deux blocs de support 17, 20 sont en outre solidaires d'une plaque 30 comportant un trou taraudé (fileté 31 dans lequel est engagée une vis 32 dont le tête 33 vient en butée sur la plaque de butée 12 solidaire du bâti pour permettre le réglage de la position correcte de la grille 9, lorsqu'elle est insérée dans la trajectoire du faisceau sous l'effet du ressort de rappel 16 ancré au bâti et tirant sur la chaîne 14.

Le moteur 28 entraîne en rotation par l'intermédiaire d'un coupleur 34, l'arbre fileté 25 dans l'un ou dans l'autre sens, pour permettre, par une translation dans les sens de la double flèche 35, le réglage correct de la position des fentes 10 de la grille 9 primaire par rapport aux centres des collimateurs secondaires 7, avant chaque mesure en mode de résolution spatiale. Le guidage de cette translation est effectué, d'une part, par les deux paliers 18 et 22 portant les axes 11 et 21 de façon axialement déplaçable et, d'autre part, par la tête de vis 33 qui est maintenue en butée sur la plaque 12 par le ressort de rappel 16 agissant sur le pignon 13 par l'intermédiaire de la chaîne 14. Les détecteurs 8 donnent un maximum de signal à la sortie lorsque la grille 9 est correctement positionnée.

Lorsque l'on désire effectuer un examen en mode de résolution en densité, on escamote la grille 9 en appliquant une tension d'alimentation continue à l'enroulement 19 de l'électroaimant de sorte qu'il attire le noyau plongeur 15.

Un perfectionnement du dispositif de collimation primaire qui permet de produire des pinceaux fins de largeur réglable, est réalisable à l'aide de deux grilles primaires superposées à ouvertures relativement larges, escamotables ou non, déplaçables en translation en sens contraires de façon à ajuster la largeur des fentes et des pinceaux, par conséquent, pour obtenir la résolution spatiale désirée.

On notera ici, que le traitement de l'information constituée par les signaux électriques issus des détecteurs pour la reconstitution de l'image de la coupe à l'aide d'un ordinateur, est adapté au mode de fonctionnement (résolution en densité ou spatiale) choisi par l'opérateur.

**Revendications**

1. Procédé de tomographie axiale transverse dans lequel une source de rayons (1) émet un faisceau (2) ou une pluralité de faisceaux en éventail obtenus à l'aide d'un collimateur primaire (4, 5) vers le corps à examiner (3), les rayons transmis étant ensuite recueillis, à travers une première rangée de collimateurs secondaires (7) à large ouverture, par une pluralité de détecteurs (8) formant une seconde rangée, caractérisé en ce que, pour obtenir dans certains cas une image d'une section du corps (3) présentant une résolution spatiale accrue, on interpose entre la source (1) et le corps (3) une grille primaire amovible (9), munie de fentes (10) laissant passer chacune un pinceau fin de largeur inférieure à celle définie par les ouvertures des collimateurs primaires et/ou secondaires (7) et, pour obtenir dans d'autres cas une image présentant une résolution en densité accrue, on escamote la grille amovible (9) de la trajectoire du ou des faisceaux en éventail (2).

2. Appareil de tomographie axiale transverse pour la mise en oeuvre du procédé suivant la revendication 1, caractérisé en ce qu'il comporte, en amont du corps à examiner (3), une grille primaire (9) escamotable pouvant être insérée et extraite de la trajectoire du ou des faisceaux en éventail, la grille étant munie de fentes (10) laissant passer une pluralité de pinceaux fins de rayons X dont la largeur est inférieure à celle obtenue à l'aide des collimateurs primaires et/ou secondaires (7).

3. Appareil suivant la revendication 2, caractérisé en ce que la grille primaire (9) est montée sur le bâti de celui-ci de façon pivotable autour d'un axe (11) pour son escamotage et déplaçable parallèlement (35) à cet axe pour son réglage en position par rapport aux collimateurs secondaires (7), les deux mouvements étant respectivement indépendamment motorisés (19 et 28).

**Claims**

1. Method for axial transverse tomography in which a radiation source (1) emits one or more fan-shaped beams (2) obtained with the aid of a primary collimator (4, 5) against a body (3) to be investigated and the transmitted rays are then captured through a first row of secondary collimators (7) of large aperture by a plurality of detectors (8) which form a second row, characterized in that, for obtaining in certain cases an image of a section of the body (3) with increased spatial resolution between the radiation source (1) and the body (3) a removable primary grating (9) is inserted which is provided with slots (10) which each allow to pass a fine beam having a width which is smaller than the width defined by the apertures of the primary collimator and/or the secondary collimators (7) and that, for obtaining in other cases an image with increased density resolution, the removable grating (9) is taken out of the trajectory of the fan-shaped beam or beams (2).

2. Apparatus for axial transverse tomography for carrying out the method according to claim 1, characterized by a removable primary grating (9) which is disposed in front of the body (3) to be investigated and which can be brought into the trajectory of the fan-shaped beam or beams (2) and taken out of said trajectory and which is provided with slots (10) which allow to pass a plurality of fine X-ray beams whose width is smaller than the width of the beams obtained by means of the primary collimator and/or the secondary collimators (7).

3. Apparatus according to claim 2, characterized in that the primary grating (9) is mounted on the frame of the apparatus pivotably about an axis (11) for removal from the path and displaceable (35) parallel to said axis for positioning with respect to the secondary collimators (7), the two movements being motor-driven (19 and 28) independently of each other.

## Patentansprüche

1. Verfahren zur axialen transversalen Tomographie, bei dem eine Strahlenquelle (1) ein oder mehrere, mit Hilfe eines Primärkollimators (4, 5) erhaltene fächerförmige Strahlenbündel (2) gegen einen zu untersuchenden Körper (3) abstrahlt und die durchgedrungenen Strahlen dann durch eine erste Reihe von Sekundärkollimatoren (7) mit großer Apertur hindurch von mehreren Detektoren (8), die eine zweite Reihe bilden, aufgefangen werden, dadurch gekennzeichnet, daß zwischen die Strahlenquelle (1) und den Körper (3) ein entfernbares Primärgitter (9) eingefügt wird, das mit Schlitzen (10) versehen ist, die jeweils ein feines Strahlenbündel mit einer Breite durchlassen, die kleiner als die von den Öffnungen des Primärkollimators und/oder der Sekundärkollimatoren (7) definierten Breite ist, damit in gewissen Fällen eine Abbildung eines Schnitts des Körpers (3) mit gesteigerter räumlicher Auflösung erhalten wird, und daß das entfernbare Gitter (9) aus der Bahn des oder der fächerförmigen Strahlenbündel (2) herausgenommen wird, damit in anderen Fällen eine Abbildung mit gesteigerter Dichteauflösung erhalten wird.

2. Einrichtung für die axiale transversale Tomographie zur Durchführung des Verfahrens nach Anspruch 1, gekennzeichnet durch ein vor dem zu untersuchenden Körper (3) befindliches, entfernbares Primärgitter (9), das in die Bahn des oder der fächerförmigen Strahlenbündel (2) gebracht und aus dieser Bahn herausgenommen werden kann und das mit Schlitzen (10) versehen ist, die mehrere feine Röntgenstrahlenbündel durchlassen, deren Breite kleiner als die Breite der mittels des Primärkollimators und/oder der Sekundärkollimatoren (7) erhaltenen Strahlenbündel ist.

3. Einrichtung nach Anspruch 2, dadurch gekennzeichnet, daß das Primärgitter (9) am Gestell der Einrichtung zum Herausnehmen aus der Bahn um eine Achse (11) schwenkbar und zum Positionieren bezüglich der Sekundärkollimatoren (7) zu dieser Achse parallel verschiebbar (35) angebracht ist, wobei die zwei Bewegungen jeweils unabhängig voneinander motorgetrieben (19 und 28) sind.

**0 001 523**

**Fig_1**

**Fig_2**

0 001 523

Fig. 2